# EUROPÄISCHE PATENTSCHRIFT

(11) **EP 0 575 852 B1**
(45) Veröffentlichungstag und Bekanntmachung des Hinweises auf die Patenterteilung: **09.10.1996**
(21) Anmeldenummer: 93109467.6
(22) Anmeldetag: 14.06.1993
(51) Int. Cl.: C07C 205/06, C07C 201/08

(54) **Verfahren zur Herstellung von Dinitro-polyalkylenbenzolen**
Process for preparing dinitro-polyalkyl-benzenes
Procédé pour la préparation de dinitropolyalcoylbenzènes

(30) Priorität: 24.06.1992 DE 4220565
(43) Veröffentlichungstag der Anmeldung: 29.12.1993
(73) Patentinhaber: BAYER AG, 51368 Leverkusen (DE)
(72) Erfinder: Blank, Heinz-Ulrich, Dr., D-5068 Odenthal-Glöbusch (DE); König, Bernd-Michael, Dr., D-4000 Düsseldorf (DE)

(56) Entgegenhaltungen:
- DE-A- 3 317 649
- DE-B- 1 105 860
- DE-C- 679 279
- US-A- 1 892 128
- US-A- 2 864 871
- US-A- 3 153 099
- HOUBEN-WEYL "Methoden der organischen Chemie", 4. Auflage, Band X/1: "Stick- stoffverbindungen 1" 1971, GEORG THIEME VERLAG, Stuttgart Seiten 532-535
- THE JOURNAL OF THE AMERICAN CHEMICAL SOCIETY, Band LXV, Juli-Dezember 1943, Easton A. NEWTON "Polyisopropyl- benzenes. II. Nitro and Amino Derivatives" Seiten 2434-2439

## Beschreibung

Die vorliegende Erfindung betrifft ein Verfahren zur Dinitrierung von Polyalkylbenzolen. Hierbei wird in einem Reaktionsmedium von H₂SO₄ mit HNO₃ nitriert. Für dieses Verfahren ist kein organisches Lösungsmittel erforderlich.

Ein wichtiger Vertreter von Dinitro-polyalkylbenzolen ist das 2,4-Dinitro-1,3,5-triisopropylbenzol, welches ein wichtiges Vorprodukt in der Chemie der Kunststoffe darstellt.

1,3,5-Triisopropylbenzol läßt sich im System HNO₃/Acetanhydrid in einfacher Weise mononitrieren; in ebenfalls glatter Reaktion wird 2,4,6-Trinitro-1,3,5-triisopropylbenzol durch Zugabe von 1,3,5-Triisopropylbenzol zu einem H₂SO₄/HNO₃-Gemisch erhalten (J.Am.Chem.Soc. 65 (1943), 2434). Nach den beschriebenen Verfahrensvarianten läßt sich ein Dinitro-triisopropylbenzol nicht herstellen. Weitere Polyalkylbenzole, wie 1,3-Diisopropylbenzol, 1,4-Diisopropylbenzol, 1,3-Dimethyl-5-tert.-butylbenzol und 1,3-Dimethyl-5-dimethylpropyl-benzol, können dadurch nitriert werden, daß das jeweilige Edukt mit konzentrierter Schwefelsäure gemeinsam vorgelegt wird und konzentrierte HNO₃ zudosiert wird (J. Am. Chem. Soc., loc.cit.; DE-OS 3 317 649). Im Falle der Dinitrierung erhält man hierbei ungenügende Ergebnisse.

Die beschriebenen Methoden eignen sich demnach für die Mononitrierung von Polyalkylbenzolen. Die erwähnte Trinitrierung von 1,3,5-Triisopropylbenzol gelang nur durch starke Temperaturerhöhung bis nahe 100°C, wobei das nitrierte Edukt schmolz und im Verlaufe der weiteren Nitrierung zu einer pastösen Masse wurde. Die auf die beschriebene Weise erreichbaren Ausbeuten und besonders die erreichbaren Reinheiten sind ungenügend. Dies muß im allgemeinen dem Umstand zugeschrieben werden, daß ein bei der Weiternitrierung erhaltenes Dinitro- oder Trinitroprodukt noch nicht umgesetztes Mononitroprodukt umhüllt und so dessen weitere Nitrierung verhindert.

Es hat daher bereits Versuche gegeben, die Nitrierung beispielsweise von 1,3,5-Triisopropylbenzol statt im Zweiphasensystem Fest/Flüssig (Mononitro-Triisopropylbenzol/Säuren) im Zweiphasensystem Flüssig/Flüssig (Edukt im organischen Lösungsmittel/Säuren) durchzuführen. Als geeignete Lösungsmittel sind hierzu aliphatische Kohlenwasserstoffe (Gemische mit einem Siedepunktsbereich von 170 bis 190°C) beschrieben worden (DE-PS 11 05 860). Eine solche Verfahrensweise ist stets mit der Notwendigkeit eines Lösungsmittel-Kreislaufs und dessen Handhabung verbunden; ferner müssen arbeitshygienische und sicherheitstechnische Aspekte (Brandgefahr) berücksichtigt werden.

Es hat sich nun gezeigt, daß eine hochselektive Dinitrierung von Polyalkylbenzolen möglich ist, wenn man ohne Verwendung eines organischen Lösungsmittels nur in H₂SO₄ als Reaktionsmedium arbeitet und das Edukt und die HNO₃ simultan in dieses Medium hineindosiert, wobei die weiteren unten angegebenen Parameter eingehalten werden.

Gemäß den US-PS'en 2 864 871 und 3 153 099 ist es bekannt, Dinitrotetraalkylbenzole durch Dinitrierung von Tetraalkylbenzolen herzustellen. Die Arbeitsweise der US-PS 2 864 871 sieht vor, daß man Salpetersäure vorlegt und pulverisiertes Tetraalkylbenzol portionsweise zufügt. Es wird dabei keine Schwefelsäure verwendet. Die Arbeitsweise der US-PS 3 153 099 sieht vor, daß man Tetraalkylbenzol und Schwefelsäure vorlegt und dann Nitriersäure zudosiert. Die Zugabe von Nitrieragens und Substrat erfolgt nicht simultan. Diese Arbeitsweisen können die vorliegende Erfindung nicht nahelegen, weil erfindungsgemäß Dinitro-dialkyl-und -trialkylbenzole hergestellt werden, wobei man das Substrat und das Nitrieragens simultan eindosiert. Im Gegensatz zu den Verfahren der US-PS'en, wo man mit beliebigen Überschüssen an Salpetersäure arbeiten kann ohne Übernitrierung befürchten zu müssen (alle anderen Kern-C-Atome sind besetzt) sind erfindungs-gemäß die im Hauptanspruch angegebenen Parameter einzuhalten, um selektiv die Dinitroverbindungen zu erhalten. Bei den erfindungsgemäß einzusetzenden Substraten können auch Tri- und gegebenenfalls Tetranitroverbindungen entstehen.

Es wurde ein Verfahren zur Herstellung von Dinitro-polyalkylbenzolen der Formel 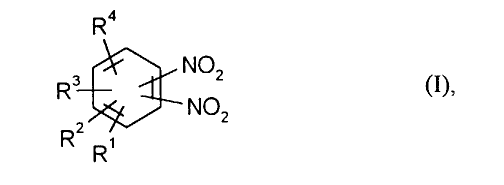 in der
- R¹ und R²: unabhängig voneinander geradkettiges oder verzweigtes C₁-C₈-Alkyl bedeuten,
- R³: für Wasserstoff steht und
- R⁴: Wasserstoff, geradkettiges oder verzweigtes C₁-C₄-Alkyl, Chlor oder Brom bedeutet,
wobei bei Abwesenheit von Chlor oder Brom die Gesamtzahl der Alkyl-C-Atome mindestens 4 beträgt, durch Nitrierung von Polyalkylbenzolen der Formel 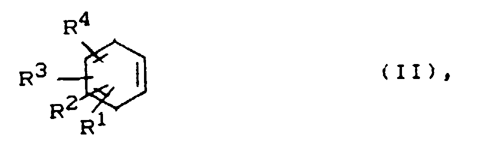 in der R¹ bis R⁴ die angegebene Bedeutung haben, wobei während der Reaktion ein Feststoff anfällt,
mit HNO₃ in Gegenwart von H₂SO₄ gefunden, das dadurch gekennzeichnet ist, daß man
a) als Reaktionsmedium H₂SO₄ ohne Mitverwendung eines organischen Lösungsmittels in einer Menge von 180 bis 1000 g, bevorzugt 200 bis 500 g, besonders bevorzugt 250 bis 350 g pro Mol des Polyalkylbenzols und mit einer Konzentration von 81 bis 96 Gew.-%, bevorzugt 85 bis 94 Gew.-%, besonders bevorzugt 89 bis 93 Gew.-%, zu mindestens 30 %, bevorzugt zu mindestens 50 % der H₂SO₄-Menge vorlegt,
b) im Bereich von 0 bis 60°C, bevorzugt 15 bis 40°C, besonders bevorzugt 20 bis 35°C, das Polyalkylbenzol, 95 bis 100 Gew.-%ige HNO₃ sowie gegebenenfalls restliche H₂SO₄ simultan so eindosiert, daß sich die zudosierten Stoffe Polyalkylbenzol und HNO₃ während der Dosierung im molaren Verhältnis von 1:2-10, bevorzugt 1:2,5-5, besonders bevorzugt 1:2,7-3,5 befinden,
c) nach der Dosierung das Reaktionsgemisch zur Nachreaktion bei 10 bis 70°C, bevorzugt 30 bis 60°C, besonders bevorzugt 40 bis 55°C hält und
d) das Dinitro-polyalkylbenzol durch Aufarbeitung gewinnt.

Geradkettiges oder verzweigtes C₁-C₈-Alkyl ist beispielsweise Methyl, Ethyl Propyl, Isopropyl, Butyl, Isobutyl, tert.-Butyl sowie die geradkettigen und verzweigten Pentyle, Hexyle, Heptyle und Octyle.

In den erfindungsgemäß einzusetzenden Polyalkylbenzolen beträgt die Gesamtzahl der C-Atome in den Alkylketten mindestens 4, in bevorzugter Weise mindestens 6.

Erfindungsgemäß einsetzbare Polyalkylbenzole sind bevorzugt solche der Formel 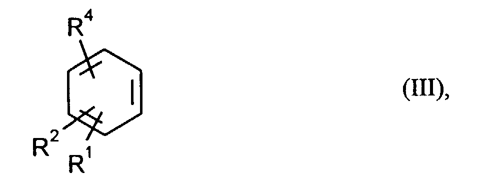 in der R¹, R² und R⁴ die oben angegebene Bedeutung haben.

Erfindungsgemäß einsetzbare Polyalkylbenzole sind besonders bevorzugt solche der Formel 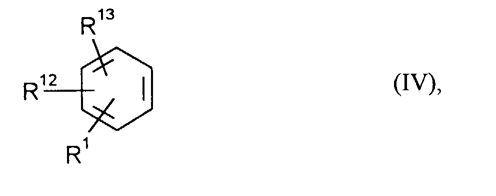 in der
- R¹: den obigen Bedeutungsumfang hat und
- R¹² und R¹³: unabhängig voneinander geradkettiges oder verzweigtes C₁-C₄-Alkyl bedeuten.
Wichtige Polyalkylbenzole für das erfindungsgemäße Verfahren sind: 1,3,5-Triisopropylbenzol, 1,2,4-Triisopropylbenzol, 1,3-Diisopropylbenzol, 1,3-Diisopropyl-5-methylbenzol, 1,3,5-Tri-t-butylbenzol, 1,2-Dimethyl-4-chlorbenzol.

Das Reaktionsmedium H₂SO₄ in der angegebenen Menge und mit der angegebenen Konzentration wird zu mindestens 50 % seiner Gesamtmenge vorgelegt. Der Rest an H₂SO₄ wird in einem solchen Falle gemeinsam mit der HNO₃ zugegeben. In bevorzugter Weise wird zur Vereinfachung des gesamten Dosierprogrammes die gesamte H₂SO₄ als Reaktionsmedium vorgelegt.

Das erfindungsgemäße Verfahren ist vor allem durch die simultane Dosierung des Polyalkylbenzols und der HNO₃ gekennzeichnet. Diese beiden Reaktionspartner haben während der Dosierung das oben angegebene molare Verhältnis.

Während der Dosierung wird das Reaktionsgemisch bei einer Temperatur im Bereich von 0 bis 60°C, bevorzugt 15 bis 40°C, besonders bevorzugt 20 bis 35°C gehalten. Hierzu ist im allgemeinen wegen der stark exothermen Reaktion eine Kühlung erforderlich. Es ist jedoch möglich, die Temperatur des Reaktionsgemisches während der Dosierung von einem Wert im unteren Teil des angegebenen Bereiches auf einen Wert im oberen Teil des angegebenen Bereiches steigen zu lassen und so die Aufwendungen für die Kühlung des Reaktionsgemisches zu verringern. Im Anschluß an die Dosierung wird das Reaktionsgemisch einer Nachreaktion unterworfen, die bei einer tendenziell höheren Temperatur stattfindet als die Dosierung. Der Temperaturbereich dieser Nachreaktion liegt im allgemeinen bei 10 bis 70°C, bevorzugt 30 bis 60°C, besonders bevorzugt 40 bis 55°C. Das beschriebene Ansteigen der Temperatur während der Dosierung macht es möglich, von der Temperatur der Dosierung unmittelbar auf die Temperatur der Nachreaktion überzugehen. Die Zeitdauer der Nachreaktion ist in einer dem Fachmann bekannten Weise von der Ansatzgröße und vom Fortschritt der Reaktion abhängig; sie kann durch einfache analytische Überprüfung von gezogenen Proben überwacht werden und wird abgebrochen, wenn ein weiterer Umsatz nicht mehr stattfindet. Die Nachreaktionszeit ist selbstverständlich dann kürzer, wenn die Dosierzeit verlängert wurde. Die Gesamtzeit für das Dosieren und die Nachreaktion wird im allgemeinen auch bei kleinen Ansätzen 3 Stunden nicht unterschreiten und auch bei großen Ansätzen 12 Stunden nicht überschreiten. Für einen Reaktionsansatz von 5 kg Gesamtmasse an Medium und Reaktionspartnern sei beispielhaft eine Dosierzeit von 3 Stunden und eine weitere Nachreaktionszeit von ebenfalls 3 Stunden genannt.

Durch das freiwerdende Reaktionswasser wird die Konzentration der eingesetzten H₂SO₄ erniedrigt; bei dieser Betrachtung bleibt die gleichfalls vorliegende HNO₃ unberücksichtigt. Die eingesetzte H₂SO₄ hat eine Konzentration von 81 bis 96 Gew.-% (Rest Wasser), bevorzugt 85 bis 94 Gew.-%, besonders bevorzugt 89 bis 93 Gew.-% und wird in einer Menge von 180 bis 1000 g, bevorzugt 200 bis 500 g, besonders bevorzugt 250 bis 350 g pro Mol des Polyalkylbenzol eingesetzt. Diese Konzentrations- und Mengenangaben sind auf die Mitverwendung einer 95 bis 100 gew.-%igen HNO₃, bevorzugt einer 98 bis 100 gew.-%igen HNO₃ ausgelegt. Selbstverständlich kann durch eine einfache Rechnung ein etwas höherer Wassergehalt bei der HNO₃ durch eine etwas höhere H₂SO₄-Konzentration im Rahmen der oben angegebenen Bereiche kompensiert werden.

In bevorzugter Weise hat die H₂SO₄ im Reaktionsgemisch nach Beendigung der Reaktion eine Konzentration von 75 bis 85 Gew.-%, bevorzugt 77 bis 84 Gew.-%, besonders bevorzugt 79 bis 83 Gew.-%, die lediglich auf das Gesamtgewicht der H₂SO₄ und des Verdünnungswassers dieser eingesetzten H₂SO₄ und des hinzugekommenen Reaktionswassers bezogen ist und die Menge der ebenfalls vorhandenen HNO₃ nicht berücksichtigt.

Im erfindungsgemäßen Verfahren fällt während der Reaktion ein Feststoff an. Dies kann das Dinitro-Endprodukt, in vielen Fällen jedoch bereits das Mononitro-Zwischenprodukt sein Die erfolgreiche Durchführung der Dinitrierung ist überraschend, da befürchtet werden müßte, daß durch die Bildung des Feststoffs noch nicht umgesetztes Material abgeschirmt und damit der Umsetzung ausgeschlossen würde.

Im Anschluß an die Nachreaktion wird das in fester Form vorliegende Dinitro-polyalkylbenzol von der Hauptmenge der Säuren (H₂SO₄ + HNO₃) abgetrennt, beispielsweise durch Filtrieren oder Zentrifugieren. Das zurückbleibende feste Produkt enthält jedoch noch anhängende und eingeschlossene restliche Säuren. Zu deren Entfernung und zu einer je nach Reinheitsanforderungen erforderlichen Erhöhung der Reinheit kann anschließend eine weitere Aufarbeitung durch Umkristallisation aus Lösung oder aus der Schmelze, durch Extraktion oder durch Aufschlämmen in Wasser erfolgen. Für das Aufschlämmen in Wasser kann zusätzlich eine geringe Menge an Alkalihydroxid oder Alkalicarbonat verwendet werden. In besonders eleganter Weise erfolgt ein Aufschlämmen in Wasser unter guter Durchmischung, wobei dem Wasser 0,01 bis 1 Gew.-%, bevorzugt 0,05 bis 0,5 Gew.-%, besonders bevorzugt 0,05 bis 0,1 Gew.-% an Dispergiermittel zugesetzt wird. Durch diese Behandlung werden auch Reste eingeschlossener Säuren wirkungsvoll entfernt. Selbstverständlich sind auch höhere Anteile an Dispergiermittel möglich; sie bringen jedoch keinen zusätzlichen Erfolg und belasten eher die Reinheit des aufgearbeiteten Produkts und erschweren die Entsorgung eines solchen Behandlungswassers. Die Art des Dispergiermittels ist unkritisch: Alle dem Fachmann bekannten kationischen, anionischen oder nichtionischen Dispergiermittel haben sich als geeignet herausgestellt.

Als Kriterium für diese Variante der Aufarbeitung wird daher vor allem der Preis des Dispergiermittels angelegt. Unter diesem Gesichtspunkt haben sich Ethoxylate und Propoxylate von Fettalkoholen, Fettsäuren und Fettsäurederivaten, sowie besonders Alkansulfonate und Alkylbenzolsulfonate als günstig herausgestellt.

Die Ausbeute an Dinitro-polyalkylbenzol erreicht mindestens 90 % der theoretischen Ausbeute, in vielen Fällen 95 % und darüber. Als typische Ausbeute für das 2,4-Dinitro-1,3,5-triisopropylbenzol seien 98 % der theoretischen Ausbeute angegeben, wobei ein solches Material 98,5 % 2,4-Dinitro-1,3,5-triisopropylbenzol, 0,4 bis 0,5 % 2-Nitro-1,3,5-triisopropylbenzol, 0,3 bis 0,4 % 2,4,6-Trinitro-1,3,5-triisopropylbenzol und weniger als 0,1 % Säuren enthält. Das erfindungsgemäße Verfahren weist gegenüber den bisherigen eine Reihe von Vorteilen auf:
- Der aufwendige Umgang mit einem Lösungsmittel entfällt; hierdurch wird die Raumausbeute beträchtlich erhöht.
- Die simultane Dosierung von Edukt und HNO₃ ergibt eine hochselektive Reaktion.
- Durch die bevorzugte Variante der Aufarbeitung mit Dispergiermittel enthaltendem Wasser entfällt auch der Gebrauch eines Lösungsmittels für eine etwaige Umkristallisation oder Extraktion und ergänzt damit den Fortfall eines Lösungsmittels während der Reaktion.
- Die hochwirksame Behandlung des Produktes mit dispergiermittelenthaltendem Wasser erlaubt eine Reduktion der Abwasserbelastung.
- Die Entfernung der Säuren vor der Nachbehandlung mit dispergiermittelenthaltendem Wasser läßt diese Säuren in einer für die üblichen H₂SO₄-Aufarbeitungsverfahren geeigneten Konzentration anfallen, so daß hierbei sowohl der größte Teil der HNO₃ als auch die gesamte H₂SO₄ wieder in den Prozeß zurückgeführt werden können.

### Beispiel

In einem 4 l-Sulfierbecher mit Bodenablaß und Ankerrührer wurden 1.292,1 g 91%ige H₂SO₄ (= 1.175,8 g 100%ig = 12 mol) vorgelegt. Zu dieser Vorlage wurden innerhalb von 3 Stunden simultan 834,1 g 98%iges Triisopropylbenzol (= 817,3 g 100%ig = 4 mol) und 771,6 g 98%iger HNO₃ (= 756,2 g 100%ig = 12 mol) eindosiert. Die Temperatur wurde hierbei bei 25°C (maximaler Anstieg bis 30°C) durch externe Kühlung gehalten. Nach etwa 2 Minuten begann festes Produkt auszufallen. Im Verlauf der Dosierung wurde die Suspension vorübergehend dicker, blieb aber einwandfrei rührbar. Anbackungen und Klumpenbildung traten nicht auf. Gegen Ende der Dosierung wurde die Korngröße des suspendierten Produkts wieder kleiner. Nach Ende der Dosierung wurde das Reaktionsgemisch innerhalb von 30 Minuten auf 50°C erwärmt, wobei eine nur geringe Entwicklung von nitrosen Gasen auftrat; es wurde 3 Stunden bei 50°C nachgerührt.

### Aufarbeitungsvariante 1

Die feine, gut bewegliche Suspension wurde in etwa 2.000 ml Eis/Wasser-Gemisch (1:1) innerhalb von 30 Sekunden abgelassen. Das verwässerte Reaktionsgemisch wurde 30 Minuten gerührt und dann innerhalb von etwa 50 Sekunden abgesaugt. Das dabei erhaltene Rohprodukt (1.512 g mit einem Säuregehalt von 9,2 Gew.-% gemäß Titration) wurde mit 1.510 ml einer 0,075 Gew.-%igen wässrigen Lösung von Alkylbenzol-Na-Sulfonat 1,5 bis 2 h bei Raumtemperatur gerührt, anschließend abgesaugt und dreimal mit je 750 ml Wasser gewaschen und an der Luft getrocknet. Das Feuchtgewicht betrug etwa 1.290 g, das Trockengewicht 1.172 g (= 98 % der theoretischen Ausbeute). Das trockene Produkt stellte fast farblose Kristalle mit einem Fp. von 135 bis 135,5°C dar. Die Analytik ergab gemäß gaschromatographischer Analyse einen Gehalt von 98,6 % an Dinitro-triisopropylbenzol, 0,4 bis 0,5 % an Nitro-triisopropylbenzol, 0,3 bis 0,4 % an Trinitro-triisopropylbenzol, 0,06 % an Wasser und 0,08 % an Restsäure (alles als Gew.-%). Nicht umgesetztes Triisopropylbenzol konnte nicht nachgewiesen werden.

### Aufarbeitungsvariante 2

Das Reaktionsgemisch wurde aus dem Sulfierbecher auf eine Glasfritte abgelassen und abgesaugt. Man erhielt 1.134 g Gebrauchtsäure mit einem Gehalt von etwa 69 Gew.-% H₂SO₄ und etwa 14,5 Gew.-% HNO₃ (Rest von ca. 16,5 % ist Wasser; der Gehalt an H₂SO₄ und H₂O entspricht einer 81%igen H₂SO₄). Der auf der Glasfritte zurückgebliebene Filterkuchen wurde mit etwa 1.000 ml Wasser gewaschen, wobei 1.090 g Waschwasser mit einem Säuregehalt von 15,5 Gew.-%, gerechnet als H₂SO₄, anfielen. Das Rohprodukt (1.520 g) wurde mit 1.520 ml einer 0,1 Gew.-%igen wässrigen Lösung von Alkylbenzol-Na-Sulfonat etwa 2 h bei Raumtemperatur gerührt, abgesaugt mit 4 x je 750 ml Wasser gewaschen und an der Luft getrocknet. Man erhielt als getrocknetes Produkt 1.178 g fast farblose Kristalle (= 98 % der theoretischen Ausbeute) mit einem Schmelzpunkt von 135 bis 135,5°C. Die gaschromatographische Analyse ergab einen Gehalt von 98,8 % an Dinitro-triisopropylbenzol, 0,5 % an Nitro-triisopropylbenzol, 0,3 % an Trinitro-triisopropylbenzol, 0,03 % an H₂O und 0,02 % an Restsäuren (alles Gew.-%). Nicht umgesetztes Triisopropylbenzol konnte nicht nachgewiesen werden.

## Patentansprüche

1. Verfahren zur Herstellung von Dinitro-polyalkylbenzolen der Formel 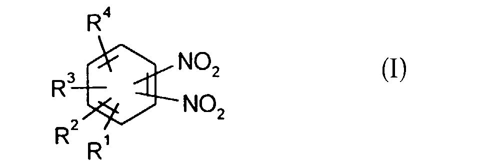 in der
R¹ und R² unabhängig voneinander geradkettiges oder verzweigtes C₁-C₈-Alkyl bedeuten,
R³ für Wasserstoff steht und
R⁴ Wasserstoff, geradkettiges oder verzweigtes C₁-C₄-Alkyl, Chlor oder Brom bedeutet,
wobei bei Abwesenheit von Chlor oder Brom die Gesamtzahl der Alkyl-C-Atome mindestens 4 beträgt,
durch Nitrierung von Polyalkylbenzolen der Formel 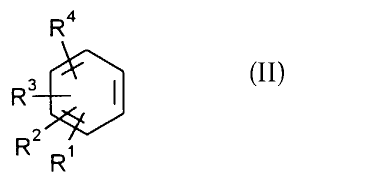 in der R¹ bis R⁴ die angegebene Bedeutung haben, wobei während der Reaktion ein Feststoff anfällt,
mit HNO₃ in Gegenwart von H₂SO₄, dadurch gekennzeichnet, daß man
a) als Reaktionsmedium H₂SO₄ ohne Mitverwendung eines organischen Lösungsmittels in einer Menge von 180 bis 1000 g pro Mol des Polyalkylbenzols und mit einer Konzentration von 81 bis 96 Gew.-% verwendet und mindestens 30 % der H₂SO₄-Menge vorlegt,
b) im Bereich von 0 bis 60°C das Polyalkylbenzol, 95 bis 100 Gew.-%ige HNO₃ sowie gegebenenfalls restliche H₂SO₄ simultan so eindosiert, daß sich die zudosierten Stoffe Polyalkylbenzol und HNO₃ sich während der Dosierung im molaren Verhältnis von 1:2-10 befinden,
c) nach der Dosierung das Gemisch zur Nachreaktion bei 10 bis 70°C hält und
d) das Dinitro-polyalkylbenzol durch Aufarbeitung gewinnt.

2. Verfahren nach Anspruch 1, dadurch gekennzeichnet, daß ein Polyalkylbenzol der Formel 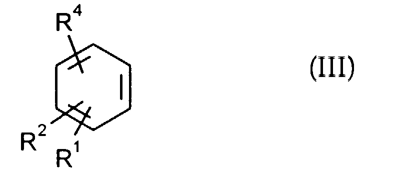 eingesetzt wird, in der
R¹ und R² unabhängig voneinander geradkettiges oder verzweigtes C₁-C₈-Alkyl bedeuten und
R⁴ für Wasserstoff und geradkettiges oder verzweigtes C₁-C₄-Alkyl steht.

3. Verfahren nach Anspruch 2, dadurch gekennzeichnet, daß ein Polyalkylbenzol der Formel 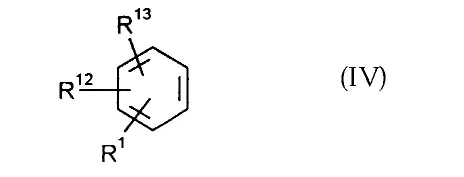 eingesetzt wird, in der
R¹ geradkettiges oder verzweigtes C₁-C₈-Alkyl bedeutet und
R¹² und R¹³ unabhängig voneinander geradkettiges oder verzweigtes C₁-C₄-Alkyl bedeuten.

4. Verfahren nach Anspruch 1, dadurch gekennzeichnet, daß ein Polyalkylbenzol mit mindestens 6 Alkyl-C-Atomen eingesetzt wird.

5. Verfahren nach Anspruch 1, dadurch gekennzeichnet, daß die gesamte H₂SO₄ vorgelegt wird.

6. Verfahren nach Anspruch 1, dadurch gekennzeichnet, daß die Reaktion so geführt wird, daß die H₂SO₄ nach Beendigung der Reaktion 75 bis 85 Gew.-% des Gesamtgewichts von H₂SO₄ und Verdünnungs- und Reaktionswasser ausmacht.

7. Verfahren nach Anspruch 1, dadurch gekennzeichnet, daß das rohe Dinitropolyalkylbenzol nach Entfernung der Hauptmenge an H₂SO₄ und HNO₃ durch Behandlung mit einer 0,01 bis 1 Gew.-%igen wässrigen Lösung eines Dispergiermittels unter Durchmischung behandelt wird.

8. Verfahren nach Anspruch 1, dadurch gekennzeichnet, daß Dinitro-triisopropylbenzole durch Umsetzung von Triisopropylbenzolen hergestellt werden.

## Claims

1. Process for the preparation of dinitro-polyalkylbenzenes of the formula 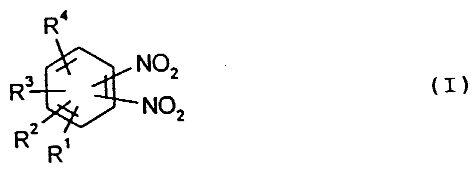 in which
R¹ and R², independently of each other, denote straight-chain or branched C₁-C₈-alkyl,
R³ represents hydrogen, and
R⁴ denotes hydrogen, straight-chain or branched C₁-C₄-alkyl, chlorine or bromine,
where, in the absence of chlorine or bromine, the total number of the alkyl C atoms is at least 4,
by nitration of polyalkylbenzenes of the formula 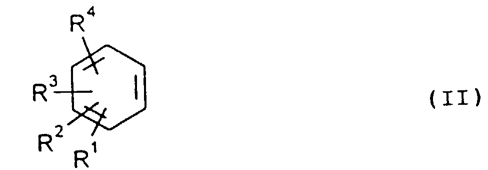 in which R¹ to R⁴ have the meaning given, a solid being produced during the reaction,
using HNO₃ in the presence of H₂SO₄, characterised in that
a) H₂SO₄ is used as reaction medium, without the accompanying use of an organic solvent, in an amount from 180 to 1000 g, per mole of polyalkylbenzene and at a concentration from 81 to 96% by weight, at least 30% of the H₂SO₄ amount is introduced,
b) the polyalkylbenzene, 95 to 100% strength by weight HNO₃ and any remaining H₂SO₄ are fed simultaneously in the range from 0 to 60°C in such a manner that the added substances polyalkylbenzene and HNO₃, during the addition, are in the molar ratio of 1:2-10,
c) after the addition, the reaction mixture is kept at 10 to 70°C for further reaction and
d) the dinitro-polyalkylbenzene is obtained by work-up.

2. Process according to Claim 1, characterised in that a polyalkylbenzene of the formula 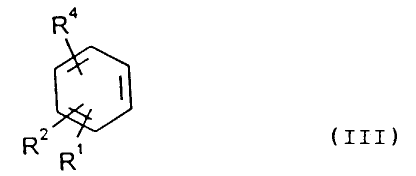 is used, in which
R¹ and R², independently of each other, denote straight-chain or branched C₁-C₈-alkyl and
R⁴ represents hydrogen or straight-chain or branched C₁-C₄-alkyl.

3. Process according to Claim 2, characterised in that a polyalkylbenzene of the formula 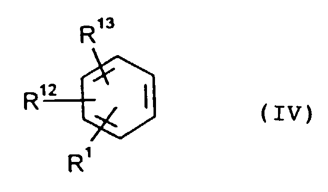 is used, in which
R¹ denotes straight-chain or branched C₁-C₈-alkyl and
R¹² and R¹³, independently of each other, denote straight-chain or branched C₁-C₄-alkyl.

4. Process according to Claim 1, characterised in that a polyalkylbenzene having at least 6 alkyl C atoms is used.

5. Process according to Claim 1, characterised in that all of the H₂SO₄ is initially introduced.

6. Process according to Claim 1, characterised in that the reaction is carried out in such a manner that the H₂SO₄, after the reaction is terminated, makes up 75 to 85% by weight of the total weight of H₂SO₄ and dilution water and water formed in the reaction.

7. Process according to Claim 1, characterised in that the crude dinitro-polyalkylbenzene, after removal of the majority of H₂SO₄ and HNO₃, is treated by treatment with a 0.01 to 1% strength by weight aqueous solution of a dispersant with mixing.

8. Process according to Claim 1, characterised in that dinitro-triisopropylbenzenes are prepared by reaction of triisopropylbenzenes.

## Revendications

1. Procédé de préparation de dinitro-polyalkylbenzènes de formule 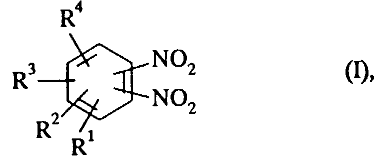 dans laquelle
R¹ et R² représentent chacun, indépendamment l'un de l'autre, un groupe alkyle en C₁-C₈ à chaîne droite ou ramifiée,
R³ représente l'hydrogène et
R⁴ représente l'hydrogène, un groupe alkyle en C₁-C₄ à chaîne droite ou ramifiée, le chlore ou le brome,
le nombre total des atomes de carbone des groupes alkyle étant d'au moins quatre en l'absence de chlore ou de brome,
par nitration des polyalkylbenzènes de formule 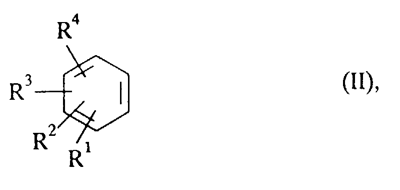 dans laquelle R¹ à R⁴ ont les significations indiquées ci-dessus, avec précipitation d'une matière solide en cours de réaction,
à l'aide de HNO₃ en présence de H₂SO₄, caractérisé en ce que
a) on utilise en tant que milieu de réaction H₂SO₄ en l'absence de solvant organique, en quantité de 180 à 1 000 g par mole du polyalkylbenzène, et à une concentration de 81 à 96 % en poids et on le met en oeuvre au départ en quantité d'au moins 30 % de la quantité totale de H₂SO₄,
b) on ajoute simultanément, dans l'intervalle de 0 à 60°C, le polyalkylbenzène, du HNO₃ à une concentration de 95 à 100 % en poids et le cas échéant le restant de H₂SO₄, en veillant à ce que, au cours de l'addition, le polyalkylbenzène et le HNO₃ ajoutés se trouvent dans un rapport molaire de 1:2 à 10,
c) après l'addition, on maintient le mélange, pour compléter la réaction, à une température de 10 à 70°C, et
d) on isole le dinitro-polyalkylbenzène.

2. Procédé selon la revendication 1, caractérisé en ce que l'on met en oeuvre un polylalkylbenzène de formule 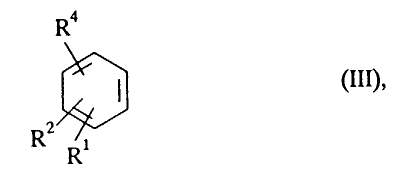 dans laquelle
R¹ et R² représentent chacun, indépendamment l'un de l'autre, un groupe alkyle en C₁-C₈ à chaîne droite ou ramifiée, et
R⁴ représente l'hydrogène ou un groupe alkyle en C₁-C₄ à chaîne droite ou ramifiée.

3. Procédé selon la revendication 2, caractérisé en ce que l'on met en oeuvre un polyalkylbenzène de formule 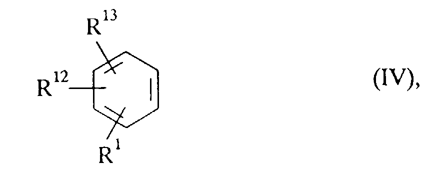 dans laquelle
R¹ représente un groupe alkyle en C₁-C₈ à chaîne droite ou ramifiée et
R¹² et R¹³ représentent chacun, indépendamment l'un de l'autre, un groupe alkyle en C₁-C₄ à chaîne droite ou ramifiée.

4. Procédé selon la revendication 1, caractérisé en ce que l'on met en oeuvre un polyalkylbenzène à au moins six atomes de carbone de groupes alkyle.

5. Procédé selon la revendication 1, caractérisé en ce que l'on met en oeuvre au départ tout le H₂SO₄.

6. Procédé selon la revendication 1, caractérisé en ce que l'on conduit la réaction de telle sorte que, après celle-ci, le H₂SO₄ représente de 75 à 85 % du poids total de H₂SO₄ et de l'eau de dilution et de réaction.

7. Procédé selon la revendication 1, caractérisé en ce que, après élimination de la plus grande partie de H₂SO₄ et de HNO₃, on traite le dinitropolyalkylènebenzène brut par une solution aqueuse contenant 0,01 à 1 % en poids d'un agent dispersant en mélangeant soigneusement.

8. Procédé selon la revendication 1, caractérisé en ce que l'on prépare des dinitro-triisopropylbenzènes par conversion de triisopropylbenzènes.
